# EUROPEAN PATENT SPECIFICATION

(11) **EP 1 264 608 B1**
(45) Date of publication and mention of the grant of the patent: **01.09.2004**
(21) Application number: 02011750.3
(22) Date of filing: 27.05.2002
(51) Int. Cl.: A61L 29/12, A61L 29/14

(54) **Composite material for medical applications, tube for medical applications and medical instrument**
Zusammengesetztes Material und Schlauch für medizinische Anwendungen sowie medizinisches Instrument
Materiau composite pour applications médicales, tube pour applications médicales et instrument médical

(30) Priority: 28.05.2001 JP 2001158627
(43) Date of publication of application: 11.12.2002
(73) Proprietor: TERUMO KABUSHIKI KAISHA, Tokyo (JP)
(72) Inventor: Kaneko, Takashi, Ashigarakami-gun, Kanagawa (JP); Tsukamoto, Hideki, Ashigarakami-gun, Kanagawa (JP)
(74) Representative: Casalonga, Axel

(56) References cited:
- EP-A- 0 573 275
- EP-A- 0 701 835
- EP-A- 0 928 611
- EP-A- 0 986 037
- US-A- 5 817 017
- PATENT ABSTRACTS OF JAPAN vol. 014, no. 531 (C-0780), 21 November 1990 (1990-11-21) & JP 02 221402 A (NIPPON STEEL CORP;OTHERS: 01), 4 September 1990 (1990-09-04)
- PATENT ABSTRACTS OF JAPAN vol. 2000, no. 01, 31 January 2000 (2000-01-31) & JP 11 293505 A (KOKAGO CORPORATION KK), 26 October 1999 (1999-10-26)
- PATENT ABSTRACTS OF JAPAN vol. 1999, no. 14, 22 December 1999 (1999-12-22) & JP 11 244399 A (NITTO DENKO CORP), 14 September 1999 (1999-09-14)

## Description

### BACKGROUND OF THE INVENTION

### 1. Field of the Invention

The present invention relates to a composite material for medical applications, to a tube for medical applications and to a medical instrument. More particularly, the present invention relates to: a medical instrument used in the field of medical cares such as cardiovascular medicine and surgery, and radiology, such as a catheter directly inserted into a human vascular system and a cardiac pacemaker (hereinafter, referred to simply as "pacemaker") indwelled in a human body for a long period of time, which has excellent resistance to electromagnetic waves; and to a tube and a composite material used in such a medical instrument.

### 2. Description of the Related Art

As a result of developments in electronics in recent years, many electronic devices utilizing electricity, electrons, radio waves and the like have come into wide use. Some of them radiate electromagnetic waves that are harmful to human bodies, other devices and the like. In recent years, among others, such electromagnetic interference has become perceived as problems.

To cope with an increasing demand for high frequency digital devices, international intensification of controls on immunity from electromagnetic wave interference (noise resistance) and study on the influences of electromagnetic waves on human bodies are being underway. However, the problems of electromagnetic wave interference in medical electronic devices (ME devices), in particular life support-related medical electronic devices, have not been solved yet, thus having become a social issue. Therefore, a specific and swift solution is desired. Should the electromagnetic interference cause any adverse influences on life support-related medical electronic devices, it will lead to a massive social disorder. Therefore, a measure for preventing such situation is becoming extremely important.

In fact, in the public transportation system, passengers are asked through announcement or advertisement with attention to influences on pacemakers to restrain from using portable phones (that is, turning the power off) when on board in vehicles. However, despite these efforts no substantial effect has been attained, thereby presenting a social issue. Pacemakers are typical examples of devices that must be free of any malfunction because their malfunction directly affects the life support system and there has been a keen demand for measures for preventing malfunctions from being caused by an electromagnetic field.

As one countermeasure for such an electromagnetic interference, various types of electromagnetic wave shielding materials have been commercially available and find utility in general industry. Main markets for electromagnetic wave shielding materials include the fields of computers, communication devices, audio-visual (AV) devices, medical electronic devices, electric measuring devices, office equipment, and automobile electric equipment. Examples of the electromagnetic wave shielding materials that have been used hitherto include materials subjected to surface treatment by electroless plating, coating with an electroconductive coating composition, metal spraying, aluminum vapor deposition, sputtering or the like to impart electroconductivity therewith; composite materials having kneaded resins with electromagnetic wave absorbers such as electroconductive plastics and ferrite; and composite materials having bonded thereon a metal foil or sheet.

Up to now, the electromagnetic wave shielding materials for general industry have been supplied mostly in the form of sheets, plates, tapes, fibers or fabrics and electromagnetic wave shielding materials having three-dimensional configuration or profile have not been put on the market yet. This is because the method of using the electromagnetic wave shielding materials is limited in applications in general industry. That is, the electromagnetic wave shielding materials for general industry are intended to be applied to the inside of the casing of an electronic device or to be wound around cables or to wrap a device, in order to attain the shielding of electromagnetic waves. Actually, users cut the electromagnetic wave shielding materials that they purchased in the form of sheet, for example, to a suitable shape and size and apply the segment to a device, wind it around the device or wrap the device with it.

However, application of such electromagnetic wave shielding materials for general industry to medical instruments such as catheters that are inserted into human bodies and directly contact the body fluid such as blood and pacemakers that are implanted into human bodies has not been realized yet. This is because the electromagnetic wave shielding materials for general industry are not supposed to be used in such a manner that they contact liquids, so that if they were used for medical instruments that are inserted into human bodies and contact the body fluid such as blood or implanted in human bodies, the problems of eluate and of corrosion would be unavoidable. In addition, the electromagnetic wave shielding materials for general industry mostly use metals that are readily ionized and eluted as ions within the human body, such as aluminum, copper and nickel, in the electric conductors or electromagnetic wave absorbers thereof. Therefore, some people could suffer from allergic reaction thereby and it is desirable to avoid the use of such metals for medical instruments. Furthermore, the difficulty in processing the electromagnetic wave shielding material into a three-dimensional molded article is another reason for the failure of its commercialization up to date.

Incidentally, many examples of malfunction of pacemakers due to electromagnetic waves have been reported. In April of 1997, the Ministry of Health and Welfare (now the Ministry of Health, Labor and Welfare) of Japan issued a guideline in which it advises pacemaker wearers that the pacemaker must be at a distance of at least 22 cm from portable phones.

However, no one can immediately tell whether or not a person being in close uses a pacemaker, so that there always exists the fear that another person being in extremely close proximity to a pacemaker wearer uses a portable phone. In particular, portable phones emits electromagnetic waves also in a standby mode so that there will emerge a very dangerous situation in a crowded space where there is no place to get away from other people and people are very close to each other, such as in a jam-packed train.

Furthermore, in addition to portable phones, there is a possibility that low frequency noises from engines, motors and the like cause malfunction of pacemakers.

As described above, under the current circumstances, pacemaker wearers are under various constraints in their daily life.

A pacemaker includes a pacemaker body and a pacemaker lead (hereinafter, sometimes referred to simply as "lead"), with the lead connecting the pacemaker body to the heart of the wearer to perform pacing and detect the action potential of the heart. The electromagnetic wave interference on pacemakers is a malfunction attributable to the lead. That is, the lead has a structure that acts as an antenna susceptible to noises and, to make the matter worse, since the action potential of a heart is as low as several millivolts (mV) the pacemakers are more susceptible to the influence of electromagnetic waves than other medical instruments. Therefore, a lead having a structure that does not act as an antenna has been sought. However, due to problems in safety, thickness, durability, and electric properties, no commercialization thereof has been made yet.

On the other hand, various means for solving the problem of electromagnetic wave interference have also been studied with respect to the pacemaker body.

For example, as described in JP 8-266647 A (the term "JP XX-XXXXXX A" as used herein means an "unexamined published Japanese patent application"), and JP 10-314318 A, means have been proposed in which the control algorithm in the pacemaker body is adapted to have a function of detecting noises and a computer program is used, which changes the operation mode to a fixed heart rate stimulation mode that does not use the active potential of heart, upon detecting noises.

However, the pacemaker wearers would feel uncomfortable as a result of the change of the operation mode into a fixed heart rate stimulation mode. In addition, in a case of a bad electromagnetic environment where the control algorithm of the pacemaker body malfunctions, there is even a danger that the pacemaker wearer will be brought into death.

Also, various means for preventing the electromagnetic wave interference of pacemakers by providing another device in the periphery of the pacemaker have been studied.

For example, JP 5-74548 A, JP 7-110352 A, JP 8-47543 A, and JP 11-33125 A describe alarm devices that inform the pacemaker wearers of the existence of dangerous electric and magnetic fields.

Surely these alarm devices are effective to some extent as countermeasures for the malfunction of pacemakers. However, because they detect dangerous electric and magnetic fields and inform pacemaker wearers of the danger, thus giving them a caution against stepping into the dangerous area, the pacemaker wearers are inevitably restricted in their action. In addition, the use of alarm devices involves a disadvantage in that the alarm causes feelings of anxiety. Furthermore, it is almost impossible for pacemaker wearers to restrict a person who carries an electronic device such as a portable phone to come up to them.

In contrast, JP 2933063 B (the term "JP XXXXXXX B" as used herein means an "Japanese patent") (JP 10-341483 A) describes a portable telephone system that includes a radio wave transmission device for informing the pacemaker wearer (physically handicapped person) of the presence of a user of a portable telephone, which is provided with a function for automatically making radio wave transmission by a communication channel impossible at the time of receiving radio waves from the radio wave transmission device.

However, this system allows the user of the portable telephone to know of the presence of the pacemaker wearer, which raises the problem that the privacy of the pacemaker wearer is violated, which will make commercialization of such system difficult.

JP 11-88300 A desbribes a device that emits jamming waves to preclude the request for a response to the receipt, which is issued from a base station of a portable phone, to prevent unconditional response transmission.

However, the use of jamming waves causes the inconvenience that the portable phone cannot be used temporarily. Also, allowing portable phone users to know that they are in an area where portable phones are usable means allowing them to know that there is a pacemaker wearer, which causes the problem of violation of privacy. Therefore, such a device is difficult to be widely accepted. That is, it is generally the mentality of the person wearing a pacemaker, who are recognized to be handicapped persons, that they do not want to be known by others as wearing pacemakers and it gives to them emotional distress if others know they wear pacemakers.

JP 2850954 B (JP 10-52506 A), Japanese utility model registration 3034951, JP 10-200289 A, JP 11-244000 A and JP 11-244399 A describe electromagnetic wave absorbing sheets or plates to be arranged outside the body for protecting pacemakers. According to the disclosure, the sheet or plate is expected to be placed near a pacemaker by applying it to the skin near a pacemaker or putting it in the breast pocket of a jacket to exhibit the effect of absorbing electromagnetic waves.

However, it is well known that the formation of an electric field shielding film must be performed such that a complete closure can be attained, and that the existence of even the slightest gap reduces the preventive effect since the electromagnetic waves will invade therethrough. Therefore, the electromagnetic wave absorbing sheets or plates described in the above publications can only serve to offer peace of mind at most, although they provide a simple and easy countermeasure. Specifically, they have the defect that although they are effective for preventing electromagnetic waves entering from the front side of the body, they are ineffective for preventing waves entering from the arms, legs, head, etc. of the body, waves entering from the sides of the body and waves entering from the back side of the body.

JP 11-293505 A and JP 11-235389 A describe clothes for shielding an electromagnetic wave, which is obtained by using an electroconductive fiber formed by plating or coating the surface thereof with silver or the like.

However, these clothes use metallic silver, nickel, copper or the like that is readily ionized on the surface of an electroconductive fiber so that the skin contacts the readily ionizable metal such as silver, resulting in that ions of silver or the like tends to be generated due to sweat or the like and some wearers may suffer from allergic reaction to metal. In fact in recent years copper, aluminum, nickel, silver, etc. have been heavily used in antimicrobial materials and the possibility of contact thereof with the human body has increased as a result. Thus, the incidence of allergic reaction to metals has been increasing. Furthermore, there have been reported cases where the use of antimicrobial catheter using silver caused allergic reaction leading to the death of patients by shock. Therefore, the use of metals that are frequently contacted by people in the daily life and have high ionic tendencies, such as silver, copper, nickel, and aluminum, is not preferable.

JP 11-178936 A describes clothes utilizing polyester cotton containing healstone that emits far-infrared rays and JP 10-37068 A describes clothes made of a fiber having dispersed therein carbon.

However, when the pacemaker wearer undresses it, for example, at the time of bathing, no preventive effect can be obtained. Accordingly, there is naturally a limitation on the protection using clothes. Further, there is the problem that conduction current generated when current directly flows in the body as a result of electrical leakage from a household electrical appliance or the like cannot be prevented.

JP 11-36131 A describes a fiber having electromagnetic wave shielding property. The fiber is applied to electromagnetic wave shielding clothes and hence has the same problems as described above. Even when using it to other applications, it could not be used as material for catheters and implants since the material from which the fiber is spun is viscose rayon (cellulose), which is a resin that swells in water to become extremely low in strength and is very susceptible to hydrolysis as is well known in the art so that its disintegration in living organism is feared.

On the other hand, as for catheters, JP 11-276484 A describes an intraperitoneal ultrasonic wave diagnostic device with less noise on image, comprising a catheter braid grounded to remove noises mixed in signal line of the ultrasonic wave catheter.

However, such a means as described above, although it is effective to shield low frequency electromagnetic waves of 1 GHz or less, is hardly effective for shielding electromagnetic waves in a frequency region above 1 GHz. Currently,-portable phones use 1.5 GHz band electromagnetic waves and use of 4 GHz band electromagnetic waves is being studied for next generation portable phones. Furthermore, with future construction of wireless LAN in hospitals in view, measures for protecting medical instruments from electromagnetic waves must be taken with ultra-broad band electromagnetic waves in mind.

EP-A-0 928 611 teaches a medical powder comprising a base particle of a ferromagnetic metal having thereon a coating, wherin at least the outside of said coating comprises a bioinert substance.

US-A-5 817 017 discloses catheters having distributed (iron) particles therein or thereon said particles optionally being coated with a polymer, e.g. a cellulose ester.

EP-A-0 701 835 (column 3) discloses a catheter having an inner layer with magnetic particles that are harmful to man. A coating is applied to the inner layer to prevent direct contact between the harmful material and the body fluids of the patient.

Finally, in EP-A-0 573 275 there is disclosed an implantable pacing lead with a porous coating made of spherical or spheroidal conductive particles (e.g. platinum, palladium) coated with a layer of nonmetallic material (e.g. titanium oxide). The lead includes an electrode for use with a cardiac pacemaker.

Having examined the prior art references centered on pacemakers as discussed above, no measure for protecting the body of a medical instrument typified by a pacemaker or catheter, which directly contacts blood or the like, from electromagnetic waves has been put into practical use.

### SUMMARY OF THE INVENTION

Therefore, an object of the present invention is to provide a composite material for medical applications and a tube for medical applications that are used in a living organism in a state where they directly contact a body fluid and prevents electromagnetic wave interference, and a medical instrument that causes no electromagnetic wave interference. By extension, an object of the present invention is to provide a countermeasure for electromagnetic waves of a medical instrument itself such as pacemaker, catheter or the like, and to provide the user of such a medical instrument with safety and peace of mind.

As a result of extensive studies with a view to solving the above-mentioned problems, the inventors of the present invention have completed the present invention by making effective use of the precision multilayer technology, that is a technology for forming a multilayer by superposing thin materials with different properties.

Therefore, the present invention provides a composite material for medical applications, comprising an outside contact layer comprised of a biocompatible material and a functional layer containing metallic powder having electromagnetic wave absorbing property, wherein each particle of the metallic powder has a flat shape.

The composite material for medical applications of the present invention ensures biocompatibility by provision of the outside contact layer so that it can be used for medical instruments to be used in a living organism in a state where they directly contact a body fluid. In addition it absorbs electromagnetic waves by provision of the functional layer so that it can protect the medical instrument against electromagnetic wave interference. The composite material for medical applications of the present invention may be provided in the form of a tube, plate, film, fiber, nonwoven fabric, cloth or the like, depending on its usage.

The present invention also provides a tube for medical applications comprising an outermost layer and at least one inner layer, wherein the outermost layer is an outside contact layer comprised of a biocompatible material and the at least one inner layer has at least one functional layer containing metallic powder having electromagnetic wave absorbing property, wherein each particle of the metallic powder has a flat shape.

The tube for medical applications of the present invention can be suitably used for catheters, pacemaker leads and the like since its outermost layer is an outside contact layer comprised of a biocompatible material and prevent catheters, pacemakers or the like from electromagnetic wave interference since at least one inner layer has electromagnetic wave absorbing property.

Furthermore, the present invention provides a medical instrument comprising a tube to be indwelled in a living organism and an electric signal transmitter inserted in the inside of the tube, wherein the tube comprises at least a functional layer containing metallic powder having electromagnetic wave absorbing property, wherein each particles of the metallic powder has a flat shape.

Usually, catheters or electrode leads tend to function as antennae since they are long and thin, but the medical instrument of the present invention is susceptible to no electromagnetic wave interference when used as a catheter or electrode lead.

According to one preferred aspect of the above-mentioned medical instrument, the tube comprises an outermost layer comprised of a biocompatible material.

In this aspect, a medical instrument is obtained that can be used in a living organism in a state where it directly contacts a body fluid.

According to another preferred aspect of the above-mentioned medical instrument, the tube comprises an electromagnetic wave shield layer comprised of an electroconductive material or a soft magnetic material.

In this aspect, due to provision of the functional layer containing metallic powder having electromagnetic wave absorbing property, the medical instrument is protected against electromagnetic wave interference occurring due to the electromagnetic waves in an ultra-high frequency region. In addition, due to provision of the electromagnetic wave shield layer comprised of an electroconductive material or a soft magnetic material, the medical instrument is protected against the electromagnetic wave interference occurring due to electromagnetic waves from low frequency to high frequency range.

According to one preferred aspect of each of the above-mentioned composite material for medical applications, the tube for medical applications, and the medical instrument, the metallic powder is comprised of a soft magnetic material. When the metallic powder is comprised of a soft magnetic material, because the soft magnetic material has high magnetic permeability, it generates high magnetization against external magnetic field and relatively readily absorbs the electromagnetic waves by converting them into thermal energy or the like.

According to another preferred aspect of each of the above-mentioned composite material for medical applications, the tube for medical applications, and the medical instrument, the metallic powder is comprised of an Fe-Al-Si alloy or an Fe-Cu-Nb-Si-B alloy. The metallic powder comprised of an Fe-Al-Si alloy or an Fe-Cu-Nb-Si-B alloy has high magnetic density and low coercive force, thereby providing excellent absorbing property at high frequencies.

In each of the above-mentioned composite materials for medical applications, tube for medical applications, and medical instrument, the particles of metallic powder have a flat shape. The particles of metallic powder of a flat shape can be oriented in a certain direction so that the orientation of magnetization can be aligned. Further, arranging the metallic powder in a laminated structure lowers the probability of gaps being formed between the particles of the metallic powder so that a fallen-leaves effect or a labyrinth effect can be expected. Therefore, the electromagnetic wave absorbing property is further enhanced.

### BRIEF DESCRIPTION OF THE DRAWINGS

FIG. 1 is a schematic cross-sectional view showing one example of a tube for medical applications of the present invention;
FIG. 2 is a schematic cross-sectional side view showing one example of a tube for medical applications of the present invention;
FIG. 3 is a schematic diagram showing the construction of a pacemaker;
FIG. 4 is a schematic partial cross-section view of the pacemaker shown in FIG. 3 taken along the line A-A; and
FIG. 5 is a schematic view illustrating the state where a pacemaker is implanted in a human body.

### DETAILED DESCRIPTION OF THE PREFERRED EMBODIMENTS

Hereinafter, preferred embodiments of the present invention will be described with reference to the attached drawings. However, the present invention should not be limited to these embodiments.

The present invention relates to a composite material for medical applications comprising an outside contact layer comprised of a biocompatible material and a functional layer containing metallic powder having electromagnetic wave absorbing property wherein each particle of the metallic powder has a flat shape. Also, the present invention relates to a tube for medical applications comprising an outermost layer and at least one inner layer, wherein the outermost layer is an outside contact layer comprised of a biocompatible material and the at least one inner layer has a functional layer containing metallic powder having electromagnetic wave absorbing property wherein each particle of the metallic powder has a flat shape.

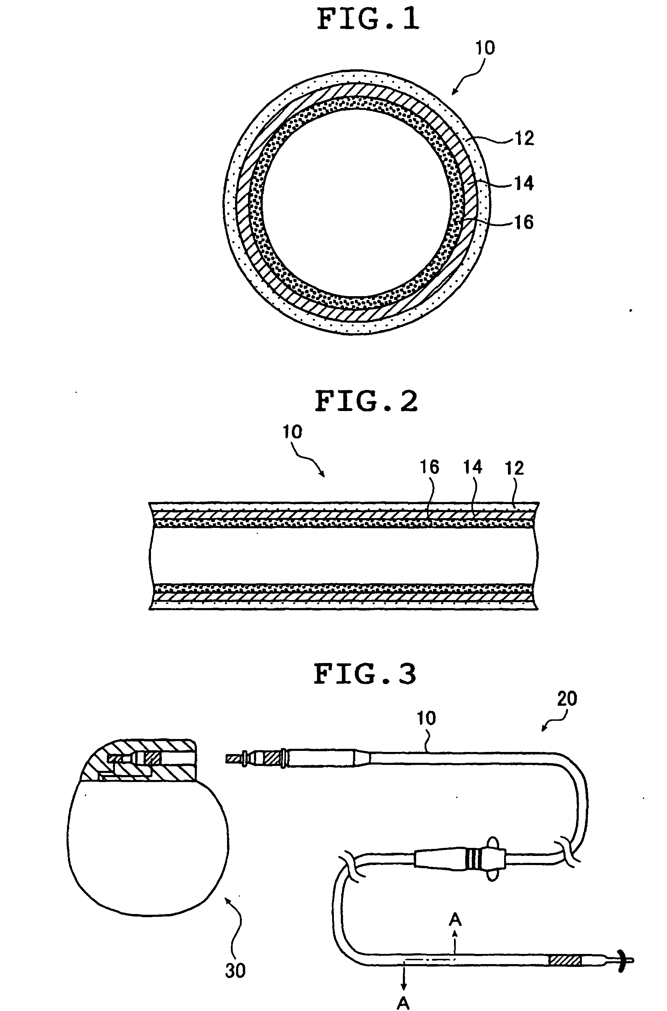

FIGS. 1 and 2 are a schematic cross-sectional view and schematic cross-sectional side view, respectively, showing one example of a tube for medical applications of the present invention.

As shown in FIG. 1, a tube 10 for medical applications comprises an outermost layer 12, which is an outside contact layer, an intermediate layer 14 and an innermost layer 16.

The material of the outermost layer 12 in the tube 10 for medical applications is not particularly limited as far as it has biocompatibility and synthetic polymers containing no additive that will impart an effect to the biocompatibility, such as, for example, a processing aid or stabilizer can be used. Specifically, commercially available thermoplastic resins or heat shrinkable tubes containing no such additive can be suitably used.

Among them, materials of a grade for wrapping food and of a grade for medical applications are preferred since such materials generate less eluate when in use. These grades are preferable since the amount of eluates is limited to a certain standard or less according to the standards approved by the authority. Those polymers usually used in catheters or pacemaker leads, for example, polyvinyl chlorides, polyurethanes, polyurethane elastomers, ethylene/vinyl acetate copolymers, chlorinated polyethylenes, ABS, polypropylenes, polyolefins, nylons, polyesters, polyester elastomers, nylon elastomers, fluoro resins, silicone elastomers, olefin elastomers, mixed polymers of these may be used.

In particular, resins such as silicone resins, polycarbonate polyurethane resins, and polyethylene resins, having grade of a medical implant that is approved to have excellent biocompatibility, in particular blood compatibility are preferred.

In a case where other resins are used, in order to improve the blood compatibility, it is preferred that an antithrombotic coat using heparin, anti-platelet drug, urokinase or the like; a coating of a block copolymer resin having antithrombotic property characterized by having a micro phase separation structure such as hemastyrene, polyether nylon or the like; or the like are applied on the surface of the outermost layer 12

As the surface treatment of the outermost layer 12, any conventionally known surface treatment in addition to the above-mentioned ones may be used. For example, in a case where the tube 10 for medical applications is used as a catheter or a lead, a hydrophilic polymer coating layer may be provided thereon in order to decrease sliding resistance upon manipulating it. Whether or not to provide such a hydrophilic polymer coating layer may be decided depending on the application of the medical instrument or the like.

The inner layer of the tube 10 for medical applications is constituted by the intermediate layer 14 and the innermost layer 16 as shown in FIGs. 1 and 2.

The intermediate layer 14 is a functional layer containing metallic powder having electromagnetic wave absorbing property. The functional layer is not limited particularly as far as it contains metallic powder having electromagnetic wave absorbing property.

The metallic powder according to one preferred aspect comprises a soft magnetic material in view of the fact that it has high magnetic permeability and generates large magnetization against external magnetic field. The soft magnetic material includes, for example, the soft magnetic flat particle powder as will be described later and the soft magnetic material used in the electromagnetic wave shield layer as will be described later.

According to another preferred aspect, the metallic powder comprises an Fe-Al-Si alloy or an Fe-Cu-Nb-Si-B alloy since such alloys can be obtained relatively easily and have excellent absorbing property at high frequencies.

The shape of the particles of metallic powder is flat. Specifically, the particles have an aspect ratio of preferably 5 or more, and more preferably 10 or more. The metal powder with flat particles as compared to metallic powder with spherical particles can be aligned in a certain orientation to align the orientation of magnetization. The arrangement of the particles of the metallic powder in a laminated structure makes it difficult to form gaps between the particles of the metallic powder so that a fallen-leaves effect or labyrinth effect can be expected. Therefore, the electromagnetic wave absorbing property becomes more excellent.

As the functional layer, specifically commercially available resins having mixed therein soft magnetic metallic powder with flat particles may be used as the metallic powder having electromagnetic wave absorbing property.

Examples of the practically used soft magnetic metallic powder with flat particles include flat powders of an Fe-Cu-Nb-Si-B amorphous or nanocrystal alloy, flat powders of electromagnetic stainless steel, and flat particles of Sendust (Fe-Al-Si alloy) and these may be suitably used in the present invention. The flat particles of the Fe-Cu-Nb-Si-B nanocrystal alloy have an average particle diameter of preferably 50 µm or less, more preferably 35 µm or less and preferably 25 µm or more. The flat particles of electromagnetic stainless steel have an average particle diameter of preferably 30 µm or less, more preferably 20 µm or less and preferably 0.1 µm or more.

Among them, flat particles of FINEMET (registered trademark in Japan, produced by Hitachi Metals, Ltd.), which are made of the Fe-Cu-Nb-Si-B nanocrystal alloy and flat particles of Sendust are preferred because of their having high aspect ratios.

The innermost layer 16 is an electromagnetic wave shield layer made of an electroconductive material or a soft magnetic material.

The electroconductive material includes, for example, electroconductive polymers and electroconductive metals. Specifically examples of the electroconductive polymer include rubber sheets containing carbon black, polyacetylenes, and resins containing electroconductive fillers. Also, metal-plated films and metal-deposited films may be used. The electroconductive metal include, for example, gold, silver, copper, platinum and alloys of these metals. Also, clad materials of these metals may be used. Furthermore, stainless steel, stainless clad steel and the like may be used. One specific embodiment of the electromagnetic wave shield layer made of an electroconductive material includes, for example, an electromagnetic shield layer constituted by an electroconductive polymer and an electromagnetic wave shield layer constituted by a metal braid or a metal ribbon

As the soft magnetic material, for example, permalloy, silicon steel, and electromagnetic stainless steel are suitably used since they are excellent in workability and readily available. Also, other materials, for example, Fe amorphous alloys, Co amorphous alloys, soft ferrites, nanocrystal materials, and thin film magnetic materials, which have high magnetic permeability may be used. Further, those soft magnetic materials that have been described above to be usable in the functional layer may also be used.

Preferably, taking workability and easy availability into consideration and in expectation of causing no problems upon exposure in case of breakage of the outermost layer, stainless ribbons of SUS316L steel and of SUS304H steel used for implant materials and MP35N alloy (Co-Ni alloy) used for electrode leads are used.

The electromagnetic wave shield layer basically plays the role of reflecting input electromagnetic waves and the role of dissipating the current to the ground. Therefore, it is preferably grounded in order to dissipate the current to the ground. However, when the structure does not allow grounding, the electromagnetic wave shield layer does not have to be grounded since it reflects almost all the quantity of the input electromagnetic waves.

The materials that constitute the intermediate layer 14 and the innermost layer 16 are not particularly limited and may be used interchangeably unless they directly contact a body fluid or a chemical. Accordingly, although the above explanation was on the embodiment in which the intermediate layer 14 is a functional layer containing metallic powder having electromagnetic wave absorbing property and the innermost layer 16 is an electromagnetic wave shield layer made of an electroconductive material or a soft magnetic material, an embodiment in which the intermediate layer 14 is an electromagnetic wave shield layer made of an electroconductive material or a soft magnetic material and the innermost layer 16 is a functional layer containing metallic powder having electromagnetic wave absorbing property may also be possible. These both are preferred embodiments of the present invention.

In the tube for medical applications of the present invention, although the thickness of each layer is not particularly limited, the outermost layer has a thickness of preferably 10 to 800 µm, and more preferably 50 to 600 µm. The functional layer containing metallic powder having electromagnetic wave absorbing property has a thickness of preferably 10 to 500 µm and more preferably 25 to 150 µm. When an electromagnetic wave shield layer made of an electroconductive material or a soft magnetic material is provided, the electromagnetic wave shield layer has a thickness of preferably 10 to 200 µm and more preferably 20 to 150 µm.

The whole thickness of the tube for medical applications of the present invention (thickness of the tube) is not particularly limited but is preferably 0.03 to 1.5 mm and more preferably 0.1 to 0.8 mm.

The cross-sectional shape of the tube for medical applications of the present invention is not particularly limited and includes, for example, circles, ellipses, polygons and indefinite shapes.

The tube for medical applications shown in FIGs. 1 and 2 is of a three-layered structure. However, in the present invention, a liner layer may also be provided as the innermost layer. Further, in the tube for medical applications of the present invention, the number of inner layers is not particularly limited as far as the tube has one inner layer.

In the tube 10 for medical applications, the outermost layer 12, which is an outside contact layer that contacts an living organism when it is inserted therein, is constituted by a biocompatible material having excellent blood compatibility or the like. Further, the provision of the intermediate layer 14 that absorbs hazardous electromagnetic waves and the innermost layer 16 that reflects hazardous electromagnetic waves makes the tube less susceptible to the influence of electromagnetic waves.

In this example, the intermediate layer 14 and innermost layer 16 are made of electromagnetic wave absorbing material and electromagnetic wave shield material, respectively. This structure is intended to remove the electromagnetic interference due to electromagnetic waves having a wavelength of a broad range, since the electromagnetic wave shield material made of an electroconductive material is generally effective as a countermeasure for noises caused by electromagnetic waves in a region of from a low frequency to a high frequency at 1 GHz or less while the electromagnetic wave absorbing material is effective as a countermeasure for noises caused by electromagnetic waves of an ultra-high frequency region at 1 GHz or more. The real picture of medical environment such as a hospital is that low frequency noises from electric scalpel, motors and the like and high frequency noises from portable phones, wireless LAN and the like are inundated so that it is preferred to remove the . interference caused by electromagnetic waves of such a broad range.

The method of producing the tube for medical applications of the present invention is not particularly limited and it can be produced, for example, by coextruding the outermost layer and intermediate layer (for example, resin kneaded with flat particles of a soft magnetic metal) to produce an integrate tube. In this case, a tubular structure having a metal ribbon helically wound may be used as the inner layer.

As described above, it will be fully understood that the composite material for medical applications of the present invention can be used as the tube for medical applications of the present invention or the like and is suitable as a material for medical instruments.

The composite material for medical applications of the present invention can provide a tube for medical applications made of a novel material, being excellent in all of biocompatibility, workability and physical properties, that has never been provided by the prior art. In addition, the present invention has for the first time realized new medical instruments such as catheters and electrode leads that are less susceptible to electromagnetic wave interference.

Next, the medical instruments of the present invention will be described.

The medical instrument of the present invention comprises a tube to be indwelled in a living organism and an electric signal transmitter inserted in the inside of the tube, the tube comprising at least a functional layer containing metallic powder having electromagnetic wave absorbing property.

The medical instrument of the present invention preferably has an outermost layer comprised of a biocompatible material. That is, it is preferred that the above-mentioned tube is the tube for medical applications of the present invention.

Preferably, the medical instrument of the present invention is the one in which the above-mentioned tube comprises an electromagnetic wave shield layer comprised of an electroconductive material or a soft magnetic material and more preferably is the tube for medical applications of the present invention that comprises an electromagnetic shield layer comprised of an electroconductive material or a soft magnetic material.

The tube used in the medical instrument of the present invention is the same as the tube for medical applications of the present invention except that the outermost layer comprised of a biocompatible material is not an essential constituent element so that detailed explanation thereof will be omitted here.

In the medical instrument of the present invention, the electric signal transmitter inserted in the inside of the tube is not particularly limited and generally used electric signal transmitters may be used. Examples thereof include metal wires having excellent electroconductivity such as enamel wires and precious metal clad wires.

Usually, catheters and electrode leads are long and thin so that they tend to serve as antennas. On the contrary, the medical instrument of the present invention does not cause electromagnetic wave interference when formed into a catheter or an electrode lead since the tube has at least the functional layer containing metallic powder having electromagnetic wave absorbing property.

The term "catheter" as used herein collectively refers to a tube that connects the inside of the body to the outside the body. In the present invention, preferred examples of the catheter include an electrode catheter for mapping used in electrophysiology, a cauterization catheter used in the therapy of arrhythmia, a catheter equipped with various sensors for temperature, pressure, flow rate and so on or a laser for the therapy, a guiding catheter used for inserting a catheter into the body, an ultrasonic catheter for the diagnosis/therapy, and a high frequency heated balloon catheter used for local thermotherapy from within a body or the like. According to the present invention, a reduction in exogenous noises at the time of using catheters and a reduction in noises emitted from the catheters can be realized.

The electrode lead includes, for example, lead wires for implanted defibrillators, pacemakers, and functional electric stimulators that rebuild and aid motive function. The present invention protects these medical instruments from electromagnetic wave interference.

Then, the method of using a pacemaker lead which is one preferred aspect of the medical instrument of the present invention will be described.

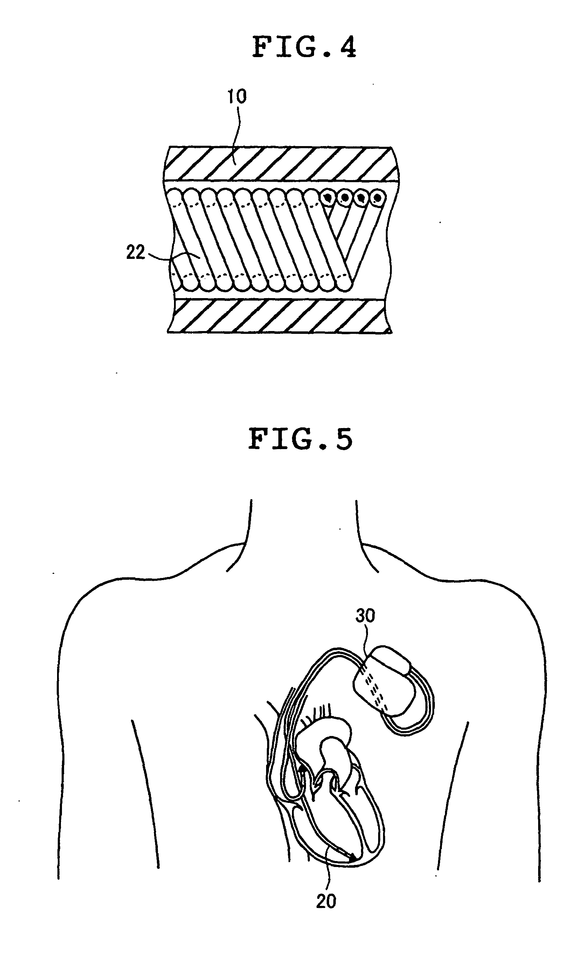

FIG. 5 is a schematic view illustrating the state in which a pacemaker with a pacemaker lead according to one preferred aspect of the medical instrument of the present invention is implanted in a human body. The pacemaker shown in FIG. 5 includes a pacemaker lead 20 and a pacemaker body 30. The pacemaker lead 20 shown in FIG. 5 is of a bipolar lead type.

The pacemaker lead 20 is connected to the pacemaker body 30 through a connector. The pacemaker body is implanted under the skin of the breast and the pacemaker lead 20 is indwelled in the heart through the subclavian vein. The pacemaker lead 20 has a proximal end portion having a connector to be connected to the pacemaker body 30 and a distal electrode portion that stimulates the heart tissue and detects electric signals of the heart action.

The pacemaker lead 20 has two basic functions of measuring weak biopotential and giving electric stimulation to the living organism by using different/indifferent electrodes and is used by indwelling thereof in the blood vessel for a long period of time.

Although explanation has been made on preferred embodiments of the present invention as described above, the present invention is not limited thereto and various alterations and modifications may be made to the present invention as far as they do not depart from the scope of the present claims.

### EXAMPLES

Hereinafter, the present invention will be described in detail by examples. However, the present invention should not be construed as being limited to these examples.

### 1. Preparation of a tube for medical applications

A tube 10 for medical applications of the present invention was prepared as follows. The tube 10 for medical applications included an outermost layer 12, an intermediate layer 14 and an innermost layer 16 as shown in FIGs. 1 and 2.

First, a metal ribbon was wound counterclockwise around a core metal of 60 cm in length and 1.7 mm in outer diameter at an angle of 45° without gaps to form a tubular monolayer coil of 48 cm in length to form an innermost layer 16. The metal ribbon was obtained by slitting a commercially available ribbon made of stainless steel SUS316L (thickness: 25 µm) to a width of 0.3 mm. Note that the direction of winding might be either clockwise or counterclockwise.

The tubular monolayer coil using a metal ribbon is served as an electromagnetic wave shield layer made of an electroconductive material and functions as a reflecting layer for electromagnetic waves. The electromagnetic wave shield layer does not need to be a monolayer coil but a multilayer multi-string flat coil contact wound in different directions may also be used. In the case of a monolayer coil, adjacent portions of the metal ribbon could form a gap therebetween upon bending. The gap was on the order of at most several millimeters and the metal ribbon portions are in a state adjacent to each other so that electromagnetic waves could not pass through the gap and be reflected or trapped by the electromagnetic wave shield layer.

In the present example, the terminals of the monolayer coil was not particularly joined, welded or bonded, or was not particularly grounded. However, they may be brazed; welded; bonded by caulking or the like; or adhered by an adhesive or the like, and they may be fixed to a terminal exposed part for grounding.

Then, over the whole length of the monolayer coil constituting the innermost layer 16 around an electromagnetic wave interference suppressor tape was wound clockwise at an angle of 45° without gaps so that the gaps between the portions of metal ribbon forming the monolayer coil could be covered to form the intermediate layer 14. The electromagnetic wave interference suppressor tape was obtained by slitting a 50 µm thick continuous sheet of an electromagnetic wave interference suppressor manufactured by Tokin Co., Ltd. (trade name "Film Impedor E50 Sheet") to a width of 1 mm. The continuous sheet was made of a resin having mixed therein flat particles of Sendust (composition: 9.5 mass% of Si, 5.5 mass% of Al, the balance Fe; average particle diameter 50 µm; aspect ratio 15; Curie temperature 500°C), which was a soft magnetic metal, as the metallic powder having electromagnetic wave absorbing property. The direction of winding might be either in the same direction as that of the monolayer coil or different therefrom.

Further, a heat shrinkable tube of silicone rubber was covered on the intermediate layer 14 and hot air was blown thereto from the outside by using a heat gun to heat shrink and bond the heat shrinkable tube to the intermediate layer 14 to form the outermost layer 12, thereby providing a three-layered structure. As the heat shrinkable tube, the one having a length before heat shrinkage of 60 cm, an inner diameter before shrinkage of 2.6 ± 0.3 mm, a thickness before shrinkage of about 0.2 mm, an inner diameter at maximum shrinkage of 1.3 mm, and a thickness after shrinkage of 0.40 ± 0.10 mm was used. Heat shrinkage tubes made of polyolefin or silicone rubber are commercially available so that ready made articles or order made articles can be obtained with ease. In addition to using a heat gun for heat shrinking, heat shrinkage tubes could be heat shrunk by charged in an oven.

Thereafter, the metal core was extracted to obtain a tube 10 for medical applications of the present invention.

The completed tube 10 for medical applications secured an inner diameter of 1.7 mm or more by the existence of the metal core. It had an outer diameter of 2.1 mm. The tube 10 for medical applications maintained the integrity of the three-layer structure by the springback by the winding of the metal ribbon used as the innermost layer 16 and the remaining contractive force of the heat shrinkable tube used as the outermost layer 12 and neither the intermediate layer 14 nor the innermost layer 16 showed a fray or projection.

In the present example, although a metal core of 60 cm in length was used, it would be easy to obtain a continuous article, for example, by using a metal core for a continuous wire so that a long scale article could be easily produced.

In the tube 10 for medical applications, the outermost layer 12 made of silicone rubber serves as a fluid contact member that contacts blood. The silicone rubber is preferable since it is excellent in biocompatibility and can be utilized as a long-term implant material. The material that constitutes the outermost layer 12 is not limited to silicone rubber but any biocompatible material may be used. In particular, it is preferred that the material is a resin that is excellent in biocompatibility and has established track record of using it as a material for catheters and the like. The outermost layer 12 does not need to be heat shrinkable but it may be formed by, for example, swelling with a solvent on the intermediate layer 14 or simply inserting a two-layer structure consisting of the innermost layer 16 and the intermediate layer 14 into the tube serving as the outermost layer 12.

It has been known that the electromagnetic wave interference suppressor used in the intermediate layer 14 absorbs electromagnetic waves in an ultra-high frequency region and suppresses eddy current, and various sheet-like articles are commercially available as products as countermeasures for electromagnetic compatibility (EMC).

However, the electromagnetic wave interference suppressors are not supposed to be used in living organisms and there is apprehension that the components could be eluted. Therefore, they cannot be used as they are as biocompatible materials.

On the other hand, the tube 10 for medical applications of the present invention can be used in living organisms since it has the outermost layer 12 made of a biocompatible material.

The innermost layer 16 is an electromagnetic shield layer. The electromagnetic wave interfering suppressor used in the above-mentioned intermediate layer 14 is designed to efficiently energy convert electromagnetic waves in the gigahertz band to remove noises so that it does not absorb electromagnetic waves of a low frequency region. Therefore, it is preferred to provide the innermost layer 16 as an electromagnetic wave shield layer that shields electromagnetic waves of a low frequency region. Note that the electromagnetic wave interference suppressor alone can have an effect on electromagnetic wave noises in a high frequency region emitted from portable phones or the like.

### 2. Preparation of pacemaker leads

By using the above-obtained tube for medical applications of the present invention, a pacemaker lead as a medical instrument of the present invention was prepared. FIG. 3 is a schematic side view showing the construction of a pacemaker that consists of the pacemaker lead and pacemaker body and FIG. 4 is a schematic partial cross-section view of the pacemaker shown in FIG. 3 along the line A-A.

The pacemaker lead 20 was prepared by inserting a resin-coated conductor coil 22 into the inside of the tube 10 of the medical applications of the present invention as shown in FIG. 4. The pacemaker lead 20 is of a monopolar lead type as shown in FIG. 3. The monopolar lead type pacemaker lead is said to be more susceptible to the influences of electromagnetic waves than the bipolar lead type pacemaker lead as shown in FIG. 5.

The pacemaker lead 20 combined with the pacemaker body 30 as shown in FIG. 3 was used in electromagnetic wave interference tests of pacemakers as described later.

### 3. Electromagnetic wave interference tests of pacemakers

A commercially available DDDR (Double chambers-Double chambers-Double rate-Responsive) type (rate-responsive type) pacemaker body of the latest type was used in a test system according to the electromagnetic wave immunity international standard IEC61000-4-3 and MIL-STD-462 to examine the relation of the occurrence of error event (electromagnetic wave interference) of a pacemaker due to electromagnetic waves and the pacemaker lead.

In a case where no pacemaker lead was connected, generation of error events was not observed even in a high frequency electric field of 400 V/m.

In a case where a commercially available pacemaker lead was connected, the pacemaker body was under the conditions of a maximum sensing sensitivity of 0.18 to 0.5 mV and the motion mode of the pacemaker body was AAI. This was the mode that was most susceptible to the influences of the electromagnetic waves. In this case, generation of error events in a band of from several tens to 800 MHz was observed in both a radiation electromagnetic field of 10 V/m and a high frequency magnetic field of 400 V/m. No influence was observed in a low frequency magnetic field of 30 kHz or less.

When the above-obtained pacemaker lead 20 was attached, the application of an electromagnetic field of an electric field strength of 400 V/m, a band of 10 kHz to 3 GHz, and a modulation frequency with variable frequency of 40 to 400 Hz, which gave a remarkable influence in a split line in case of the commercially available pacemaker resulted in no generation of error events. That is, the pacemaker lead as a medical instrument of the present invention proved to prevent malfunction of the pacemaker by direct application of intense electromagnetic wave.

As described above, the use of the tube for medical applications of the present invention as one preferred aspect of the composite material for medical applications of the present invention in catheters, pacemaker leads and the like, which have hitherto been long and thin so that they have tended to serve as antennae and thus have been susceptible to electromagnetic wave interference, results in imparting electromagnetic wave resistance thereto. In addition, the tube for medical applications as one preferred aspect of the composite material for medical applications of the present invention is designed taking the use in living organisms into full consideration so that the present invention can provide a countermeasure for avoiding electromagnetic wave interference of medical instruments that has hitherto been impossible.

Furthermore, the medical instrument of the present invention unlike the conventional medical instruments receives no electromagnetic interference so that it can be suitably used as a catheter, pacemaker lead or the like.

## Claims

1. A composite material for medical applications, comprising an outside contact layer comprised of a biocompatible material and a functional layer containing metallic powder having electromagnetic wave absorbing property, wherein each particle of the metallic powder has a flat shape.

2. A composite material for medical applications according to claim 1, wherein the metallic powder comprises a soft magnetic material.

3. A composite material for medical applications according to claim 1, wherein the metallic powder comprises an Fe-Al-Si alloy or an Fe-Cu-Nb-Si-B alloy.

4. A tube for medical applications comprising an outermost layer and at least one inner layer, wherein the outermost layer is an outside contact layer comprised of a biocompatible material and the at least one inner layer has at least one functional layer containing metallic powder having electromagnetic wave absorbing property, wherein each particle of the metallic powder has a flat shape.

5. A tube for medical applications according to claim 4, wherein the metallic powder comprises a soft magnetic material.

6. A tube for medical applications according to claim 4, wherein the metallic powder comprises an Fe-Al-Si alloy or an Fe-Cu-Nb-Si-B alloy.

7. A medical instrument comprising a tube to be indwelled in a living organism and an electric signal transmitter inserted in the inside of the tube, wherein the tube comprises at least a functional layer containing metallic powder having electromagnetic wave absorbing property, wherein each particle of the metallic powder has a flat shape.

8. A medical instrument according to claim 7, wherein the tube comprises an outermost layer comprised of a biocompatible material.

9. A medical instrument according to claim 7 or 8, wherein the tube comprises an electromagnetic wave shield layer comprised of an electroconductive material or a soft magnetic material.

10. A medical instrument according to any one of claims 7 to 9, wherein the metallic powder comprises a soft magnetic material.

11. A medical instrument according to any one of claims 7 to 9, wherein the metallic powder comprises an Fe-Al-Si alloy or. an Fe-Cu-Nb-Si-B alloy.

## Patentansprüche

1. Verbundmaterial für medizinische Anwendungen, umfassend eine Außenseiten-Kontaktschicht, bestehend aus einem bioverträglichen Material, und eine funktionelle Schicht, enthaltend metallisches Pulver, das elektromagnetische Wellen absorbierende Eigenschaft besitzt, worin jedes Teilchen des metallischen Pulvers eine flache Gestalt hat.

2. Verbundmaterial für medizinische Anwendungen nach Anspruch 1, in welchem das metallische Pulver ein weiches magnetisches Material umfaßt.

3. Verbundmaterial für medizinische Anwendungen nach Anspruch 1, in welchem das metallische Pulver eine Fe-Al-Si-Legierung oder eine Fe-Cu-Nb-Si-B-Legierung umfaßt.

4. Schlauch für medizinische Anwendungen, umfassend eine am weitesten außen liegende Schicht und wenigstens eine innere Schicht, in welchem die am weitesten außen liegende Schicht eine Außenseiten-Kontaktschicht, bestehend aus einem bioverträglichen Material, ist, und die wenigstens eine innere Schicht wenigstens eine funktionelle Schicht hat, enthaltend metallisches Pulver, das elektromagnetische Wellen absorbierende Eigenschaft besitzt, worin jedes Teilchen des metallischen Pulvers eine flache Gestalt hat.

5. Schlauch für medizinische Anwendungen nach Anspruch 4, in welchem das metallische Pulver ein weiches magnetisches Material umfaßt.

6. Schlauch für medizinische Anwendungen nach Anspruch 4, in welchem das metallische Pulver eine Fe-Al-Si-Legierung oder eine Fe-Cu-Nb-Si-B-Legierung umfaßt.

7. Medizinisches Instrument, umfassend einen Schlauch, der in einen lebenden Organismus eingesetzt worden soll, und einen Transmitter für elektrische Signale, der in der Innenseite des Schlauches eingesetzt ist, in welchem der Schlauch wenigstens eine funktionelle Schicht umfaßt, die metallisches Pulver enthält, das elektrische Wellen absorbierende Eigenschaft besitzt, in welchem jedes Teilchen des metallischen Pulvers eine flache Gestalt hat.

8. Medizinisches Instrument nach Anspruch 7, in welchem der Schlauch eine am weitesten außen liegende Schicht umfaßt, die aus einem bioverträglichen Material besteht.

9. Medizinisches Instrument nach Anspruch 7 oder 8, in welchem der Schlauch eine Abschirmungsschicht für elektromagnetische Wellen umfaßt, bestehend aus einem elektrisch leitenden Material oder einem weichen magnetischen Material.

10. Medizinisches Instrument nach irgendeinem der Ansprüche 7 bis 9, in welchem das metallische Pulver ein weiches magnetisches Material umfaßt.

11. Medizinisches Instrument nach irgendeinem der Ansprüche 7 bis 9, in welchem das metallische Pulver eine Fe-Al-Si-Legierung oder eine Fe-Cu-Nb-Si-B-Legierung umfaßt.

## Revendications

1. Matériau composite pour applications médicales, comprenant une couche de contact extérieure constituée d'un matériau biocompatible et une couche fonctionnelle contenant de la poudre métallique ayant des propriétés d'absorption d'ondes électromagnétiques, dans lequel chaque particule de la poudre métallique a une forme plate.

2. Matériau composite pour applications médicales selon la revendication 1, dans lequel la poudre métallique comprend un matériau magnétique doux.

3. Matériau composite pour applications médicales selon la revendication 1, dans lequel la poudre métallique comprend un alliage Fe-Al-Si ou un alliage Fe-Cu-Nb-Si-B.

4. Tube pour applications médicales comprenant une couche la plus externe et au moins une couche intérieure, dans lequel la couche la plus externe est une couche de contact extérieure constituée d'un matériau biocompatible et ladite au moins une couche intérieure comporte au moins une couche fonctionnelle contenant de la poudre métallique ayant des propriétés d'absorption d'ondes électromagnétiques, dans lequel chaque particule de la poudre métallique a une forme plate.

5. Tube pour applications médicales selon la revendication 4, dans lequel la poudre métallique comprend un matériau magnétique doux.

6. Tube pour applications médicales selon la revendication 4, dans lequel la poudre métallique comprend un alliage Fe-Al-Si ou un alliage Fe-Cu-Nb-Si-B.

7. Instrument médical comprenant un tube destiné à être placé à demeure dans un organisme vivant et un émetteur de signaux électriques inséré à l'intérieur du tube, dans lequel le tube comprend au moins une couche fonctionnelle contenant de la poudre métallique ayant des propriétés d'absorption d'ondes électromagnétiques, dans lequel chaque particule de la poudre métallique a une forme plate.

8. Instrument médical selon la revendication 7, dans lequel le tube comprend une couche la plus externe constituée d'un matériau biocompatible.

9. Instrument médical selon la revendication 7 ou 8, dans lequel le tube comprend une couche de protection contre les ondes électromagnétiques constituée d'un matériau électroconducteur ou d'un matériau magnétique doux.

10. Instrument médical selon l'une quelconque des revendications 7 à 9, dans lequel la poudre métallique comprend un matériau magnétique doux.

11. Instrument médical selon l'une quelconque des revendications 7 à 9, dans lequel la poudre métallique comprend un alliage Fe-Al-Si ou un alliage Fe-Cu-Nb-Si-B.
